# EUROPEAN PATENT SPECIFICATION

(11) **EP 1 824 486 B1**
(45) Date of publication and mention of the grant of the patent: **18.03.2009**
(21) Application number: 05817508.4
(22) Date of filing: 06.12.2005
(51) Int. Cl.: A61K 31/496, A61P 25/00, C07D 405/12

(54) **BENZDIOXANE PIPERAZINE DERIVATIVES WITH A COMBINATION OF AFFINITY FOR DOPAMINE-D2 RECEPTORS AND SEROTONIN REUPTAKE SITES**
BEZDIOXAN-PIPERAZIN-DERIVATE MIT EINER KOMBINATION AUS AFFINITÄT FÜR DOPAMIN-D2-REZEPTOREN UND SEROTONIN-WIEDERAUFNAHME-STELLEN
DERIVES DE BENZDIOXANE PIPERAZINE PRESENTANT UNE COMBINAISON D'AFFINITE POUR LES RECEPTEURS DE LA DOPAMINE D2

(30) Priority: 07.12.2004 EP 04106361; 07.12.2004 US 633447 P
(43) Date of publication of application: 29.08.2007
(73) Proprietor: Solvay Pharmaceuticals B.V., 1381 CP Weesp (NL)
(72) Inventor: VAN HES, Roelof, c/o Solvay Pharmaceuticals BV, NL-1381 CP Weesp (NL); SMID, Pieter, c/o Solvay Pharmaceuticals BV, NL-1381 CP Weesp (NL); KRUSE, Cornelis G., c/o Solvay Pharmaceuticals BV, NL-1381 CP Weesp (NL); TULP, Martinus T.M., c/o Solvay Pharmaceuticals BV, NL-1381 CP Weesp (NL)
(74) Representative: Hogenbirk, Marijke
(86) International application number: PCT/EP2005/056503
(87) International publication number: WO 2006/061374

(56) References cited:
- EP-A- 0 376 607
- WO-A-01/14330
- WO-A-99/05140
- WO-A-20/04054972

## Description

The present invention relates to a group of novel benzdioxane piperazine derivatives with a dual mode of action: serotonin reuptake inhibition and affinity for dopamine-D₂ receptors and to methods for the preparation of these compounds. The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a di sease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament useful in the treatment of disorders in which dopamine-D₂ receptors and serotonin reuptake sites are involved, or that can be treated via manipulation of those targets.

Phenylpiperazine derivatives with a dual action as dopamine-D₂ antagonists and serotonin reuptake inhibitors are known from WO 01/014330. This combination is useful for the treatment of schizophrenia and other psychotic disorders which enables a more complete treatment of all disease symptoms (e.g. positive symptoms and negative symptoms). Tetrahydropyridin-4-yl indole derivatives with a dual action as dopamine-D₂ antagonists and serotonin reuptake inhibitors are known from WO 00/023441 and WO 00/0694 24, and a promising clinical candidate disclosed in these patent applications was further described by Van Hes et al. (Bioorganic and Medicinal Chemistry Letters, 13(3), 405-408, 2003).

Piperazinylbutylindoles, -indazolines, the corresponding 2,3-dihydro derivatives and 2-indolones were disclosed in EP 0 376 607 A1. These compounds were characterized as serotonin 5-HT_{1A} receptor ligands. In WO 99/05140, indole and 2,3-dihydroindole derivatives with a dual mechanism of action as serotonin reuptake inhibitors and 5-HT_{1A} receptor antagonists were described, whilst in WO 2004/054972 a series of N-(indolethyl-)cycloamine derivatives were described as serotonin reuptake inhibitors and activators at 5-HT_{1A} and 5-HT_{2A} receptors. In none of the latter three patent applications antagonism of dopamine-D₂ receptors was disclosed.

The goal of the present invention was to provide further compounds with a dual action as dopamine-D₂ antagonists and serotonin reuptake inhibitors.

The invention relates to a group of novel benzdioxane piperazine derivatives of the formula (1): 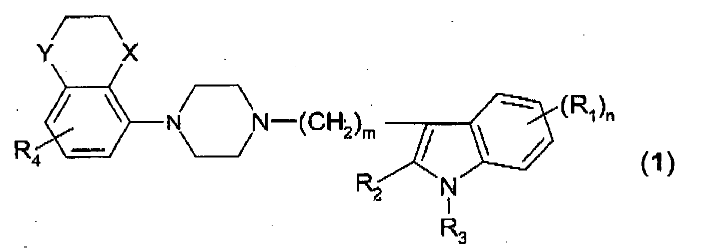 wherein:
Y is O, X is CH₂ or O, m is 1, 3, or 5, n is 1, R₂, R₃ and R₄ are hydrogen, and R₁ is hydrogen, methyl, fluorine, chlorine or methoxy,
and tautomers, stereoisomers and N-oxides thereof, as well as pharmacologically acceptable salts, hydrates and solvates of said compounds of formula (1) and its tautomers, stereoisomers and N-oxides.

In the description of the substituents the abbreviation 'alkyl(C₁₋₃)' means 'methyl, ethyl, n-propyl or isopropyl'.

N-oxides of the compounds mentioned above are in the scope of the present invention. Tertiary amines may or may not give rise to N-oxide metabolites. The extend to what N-oxidation takes place varies from trace amounts to a near quantitative conversion. N-oxides may be more active than their corresponding tertiary amines or less active. Whilst N-oxides are easily reduced to their corresponding tertiary amines by chemical means, in the human body this happens to varying degrees. Some N-oxides undergo nearly quantitative reductive conversion to the corresponding tertiary amines, in other cases the conversion is a mere trace reaction or even completely absent. (M.H. Bickel: "The pharmacology and Biochemistry of N-oxides", Pharmaco-logical Reviews, 21(4), 325 - 355, 1969).

The invention particularly relates to compounds of the general formula (1) in which: Y is O, X is CH₂ or O, m is 1, 3, or 5, n is 1, R₂, R₃ and R₄ are hydrogen, and R₁ is hydrogen, methyl, fluorine, chlorine or methoxy, and tautomers, stereoisomers and N-oxides thereof, as well as pharmacologically acceptable salts, hydrates and solvates of said compounds of formula (1) and its tautomers, stereoisomers and N-oxides.

It has been found that the compounds according to the invention show high affinity for both the dopamine D₂ receptor and the serotonin reuptake site. The compounds show activity as antagonists at dopamine D₂ receptors as they potentially antagonize apomorphine-induced climbing behaviour in mice (B. Costall et al., 'Climbing behaviour induced by apomorphine in mice: a potential model for the detection of neuroleptic activity', Eur. J. Pharmacol., 1978, 1, 39-50). The compounds also show activity as inhibitors of serotonin reuptake, as they potentiate 5-HTP induced behaviour in mice (B.L. Jacobs., 'An animal behaviour model for studying central serotonergic synapses', Life Sci., 1976, 19(6), 777 -785). The compounds are active in therapeutic models sensitive to clinically relevant antipsychotics (e.g. the conditioned avoidance response; Van der Heyden & Bradford, Behav. Brain Res., 1988, 31:61-67) and antidepres-sants or anxiolytics (e.g. suppression of stress-induced vocalization; van der Poel et al., Psychopharmacology, 1989, 97: 147-148). In contrast to clinically relevant dopamine D₂ receptor antagonists the described compounds have a low propensity to induce catalepsy in rodents and as such are likely to induce less extrapyramidal side effects than existing antipsychotic agents. The inhibitory activity of serotonin reuptake inherent in these compounds may be responsible for the therapeutic effects observed in behavioural models sensitive to either antidepressants or anxiolytics. The compounds can be used for the treatment of affections or diseases of the central nervous system caused by disturbances in either the dopaminergic or serotonergic systems, for example: aggression, anxiety disorders, autism, vertigo, depression, disturbances of cognition or memory, Parkinson's disease, and in particular schizophrenia and other psychotic disorders.

### GENERAL ASPECTS OF SYNTHESES

The compounds having formula (1) can be prepared by reaction of a compound of the formula (2): 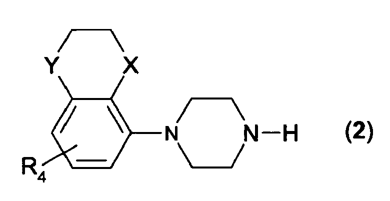 under basic conditions with a compound of the formula (3): 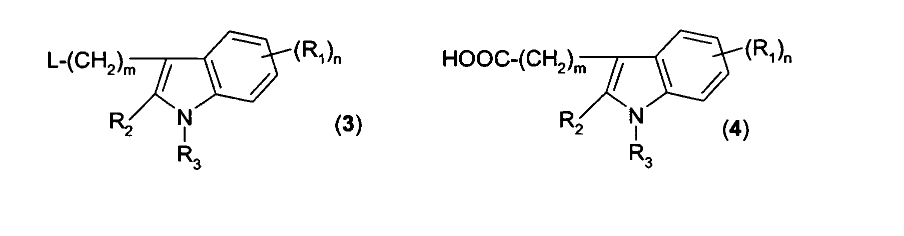 in which formulae the symbols have the meanings given above, and L is a leaving group such as a halogen atom or a mesylate group (see below: Scheme 1, Route B). An alternative route to compounds having formula (1) is depicted in Scheme 1 as Route A (*see bellow*), in which compounds (2) react with compounds (4) via a two step procedure. The starting compounds having formula (3) and (4) can be prepared according to methods known for analogues compounds, and as described for example in Organic Process Res. and Dev. 1997 (1), 300-310. The piperazine compounds having formula (2) can be obtained as described in EP 0138280, EP 0189612 and/or EP 0900792, or in an an alogous manner.

The selection of the particular synthetic procedures depends on factors known to those skilled in the art such as the compatibility of functional groups with the reagents used, the possibility to use protecting groups, catalysts, activati ng and coupling reagents and the ultimate structural features present in the final compound being prepared.

Pharmaceutically acceptable salts may be obtained using standard procedures well known in the art, for example by mixing a compound of the present invention with a suitable acid, for instance an inorganic acid such as hydrochloric acid, or with an organic acid.

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be ad ministered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification an d general knowledge in the art. In embodiments of the invention, a pharmaceutical pack or kit is provided comprising one or more containers filled with one or more of the ingredients of a pharmaceutical composition of the invention. Associated with such co ntainer(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### In vitro affinity for dopamine-D₂ receptors

Affinity of the compounds for dopamine -D₂ receptor s was determined using the receptor binding assay described by I. Creese, R. Schneider and S.H. Snyder: "[3H]-Spiroperidol labels dopamine receptors in rat pituitary and brain", Eur.J.Pharmacol., 46, 377 - 381, 1977.

### In vitro affinity for serotonin reuptake sites

Affinity of the compounds for serotonin reuptake sites was determined using the receptor binding assay described by E. Habert et al.,: "Characterisation of [3H]-paroxetine binding to rat cortical membranes", Eur.J.Pharmacol., 118, 107 - 114, 1985.

### DOSAGES

The affinity of the compounds of the invention for dopamine-D₂ receptors and serotonine reuptake sites was determined as described above. From the binding affinity measured for a given compound of formula (1), one can estimate a theoretical lowest effective dose. At a concentration of the compound equal to twice th e measured Kᵢ-value, 100% of the receptors likely will be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### TREATMENT

The term 'treatment' as used herein refers to any treatment of a mammalian, preferably human condition or disease, and includes: (1) preventing the disease or condition from occurring in a subject which may be predisposed to the disease but has not yet been diagnosed as having it, (2) inhibi ting the disease or condition, i.e., arresting its development, (3) relieving the disease or condition, i.e., causing regression of the condition, or (4) relieving the conditions caused by the disease, i.e., stopping the symptoms of the disease.

### ABBRVS

In this applications some abbreviations are used that may not be completely unambiguous for the person skilled in the art. Those are:
DCC = dicyclohexylcarbodiimide
DCM = dichloromethane
DIPEA = diisopropylethylamine
HRMS = High Resolution Mass Spectrometry
5-HTP = 5-hydroxytryptophan
OMes = -O-mesylate (one of the leaving groups)
RT = room temperature

The preparation of the compounds having formula (1) will now be described in more detail in the following Examples.

### EXAMPLES

### EXAMPLE 1: MATERIALS AND METHODS

¹H and ¹³C NMR spectra were recorded on a Bruker Avance DRX600 instrument (600 MHz), Varian UN400 instrument (400 MHz) or on a Varian VXR200 instrument (200 MHz) using DMSO-D₆ or CDCl₃ as solvents with tetramethylsilane as an internal standard. Chemical shifts are given in ppm (δ scale) downfield from tetramethylsilane. Peakshapes in the NMR spectra are indicated with the symbols 'q' (quartet), 'dq' (double quartet), 't' (triplet), 'dt' (double triplet), 'd' (doublet), 'dd' (double doublet), 's' (singlet), 'bs' (broad singlet) an d 'm' (multiplet). Flash chromatography was performed using silica gel 60 (0.040-0.063 mm, Merck). Column chromatography was performed using silica gel 60 (0.063 -0.200 mm, Merck). Mass spectra were recorded on a Micromass QTOF-2 instrument with MassLynx application software for acquisition and reconstruction of the data. Exact mass measurement was done of the quasimolecular ion [M+H] ⁺. Melting points were recorded on a Büchi B-545 melting point apparatus. Yields refer to isolated pure products.

### EXAMPLE 2: SYNTHESES OF SPECIFIC COMPOUNDS

### Compounds prepared as described in Scheme (see below) 1

**3-[4-(2,3-Dihydro-benzo[1,4]dioxin-5-yl)-piperazin-1-yl-methyl]-1*H*-indole (compound 5a).** A solution of compound **2a** (2.2 g, 8.5 mmol), indole (1 g, 8.5 mmol) and formaldehyde (37%, 0.7 ml) in ethanol (30 ml) was refluxed for 18 hours. The mixture was allowed to cool to room temperature and was concentrated *in vacuo* to give crude compound **5a** as a brown oil, which was purified by silica gel column chromatography (EtOAc/ methanol, 95/5, v/v). Compound **5a** was obtained as a white solid (850 mg, 33%); mp 85-86°C; ¹H-NMR (400 MHz, DMSO-d₆): δ 10.8 (s, 1H, NH-indole); 7.65 (d, 1H, J=8Hz, H-arom); 7.35 (d, 1H, J=8Hz, H-arom); 7.18 (d, 1H, J=2Hz, H2-indole); 7.07 (t, 1H, J=7Hz, H-arom); 6.98 (t, 1H, J=7Hz, H-arom); 6.67 (t, 1H, J=7Hz, H-arom); 6.4-6.48 (2x d, 2H, H-arom); 4.2 (bm, 4H, OCH₂CH₂O); 3.7 (bs, 2H, CH₂); 3.0, 2.55 (2x bs, 8H, piperazine). HRMS (C₂₁H₂₄N₃O₂) [M+H]⁺: found *m*/*z* 350.1899, calculated 350.1869.

### General procedure for the synthesis of compounds 5b-n.

**Route A).** To a solution of compound **2a** in dry THF (5 ml/mmol) was added 3-(w-alkyl-carboxylic acid)-*1H*-indole (compound 4), dicyclohexylcarbodiimide (DCC, **1** equivalent) and the mixture was stirred overnight under nitrogen. The reaction mixture was filtered and concentrated *in vacuo*. The residue was 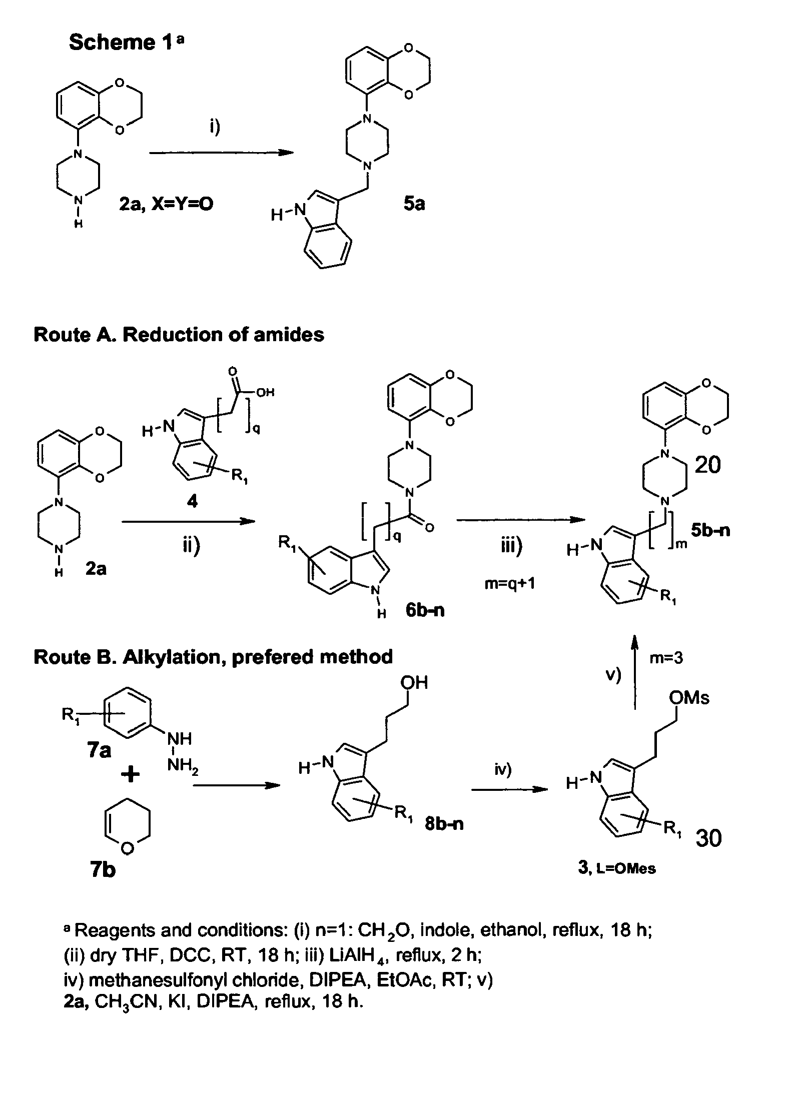 purified by silica gel column chromatography to give crude compound **6**. The resulting oil was dissolved in dry THF (5 ml/mmol), cooled to 0°C and to this mixture a solution of LiAlH₄ (1.5 equivalents) in dry THF (10 ml/mmol) was added. The reaction mixture was refluxed for 2 hours and allowed to cool to room temperature. To the cooled mixture was added 1N NaOH solution (5 ml/mmol) and the mixture was stirred for 1 hour. The mixture was extracted with DCM (2x) and the combined organic layers were washed with water and dried on MgSO₄. The crude residue was purified by silica gel column chromatography to give compound **5.** The compounds were converted to the fumaric acid salts by addition of 0.5 equivalent fumaric acid, unless stated otherwise.

**Route B).** To a solution of compound **2a** in dry acetonitril (10 ml/mmol) was added methanesulfonic acid (*1H*-indole-3-yl) alkanoic ester (compound **3,** L=OMes) (1 equivalent), potassium iodide (1 equivalent) and DIPEA (3 equivalent). The reaction mixture was refluxed for 18 hours, after which time TLC analysis revealed complete conversion of compound **3.** The mixture was concentrated *in vacuo* and the crude residue was purified by silica gel column chromatography to give compound **5.** The compounds were converted to the fumaric acid salts by addition of 0.5 equivalent fumaric acid, unless stated otherwise.

**3-{3-[4-(2,3-Dihydro-benzo[1,4]dioxin-5-yl)-piperazin-1-yl]-propyl}-1*H*-indole (compound 5b).** Compound **5b** was prepared via route B) and isolated as a white solid as the free base in a yield of 77%. TLC analysis and silica gel column chromatography: DMA 0.25, Rf 0.4; mp 150-152°C; ¹H-NMR (400 MHz, DMSO-d₆): δ 10.7 (s, 1H, NH-indole); 7.5 (d, 1H, J=8Hz, H-arom); 7.32 (d, 1H, J=8Hz, H-arom); 7.06 (d, 1H, J=2Hz, H2-indole); 7.04 (t, 1H, J=7Hz, H-arom); 6.95 (t, 1H, J=7Hz, H-arom); 6.7 (t, 1H, J=8Hz, H-arom); 6.4-6.5 (2x dd, 2H, J=2Hz, J=8Hz, H-arom); 4.2 (bm, 4H, OCH₂CH₂O); 3.0 (bs, 4H, piperazine); 2.72 (t, 2H, CH₂); 2.55 (bs, 4H, piperazin); 2.4 (t, 2H, CH₂); 1.85 (q, 2H, CH₂); ¹³C-NMR (DMSO-d₆): 144.1, 141.9, 136.6, 136.4, 127.5, 114.8 (6x C-quart); 122.7, 121.0, 120.5, 118.6, 118.3, 111.6, 111.3, 110.4 (8xCH-arom); 64.1, 64.0 (2x C-O); 50.5, 53.4 (C-piperazin); 58.0, 27.5, 22.8 (3xCH₂-propyl).

**3-{5-[4-(2,3-Dihydro-benzo[1,4]dioxin-5-yl)-piperazin-1-yl]-pentyl}-1*H*-indole (compound 5c) 2.5-fumaric acid salt.** Compound 5c was prepared via route A. Compound **2** was converted to compound 5c in a yield of 45%. TLC analysis and silica gel column chromatography: eluent DMA 0.5, Rf 0.5. Compo und **5c** gave a white solid with 3 equivalents of fumaric acid. ¹H-NMR (400 MHz, DMSO-d₆): δ 10.6 (s, 1H, NH-indole); 7.48 (d, 1H, J=8Hz, H-arom); 7.32 (d, 1H, J=8Hz, H-arom); 7.04 (m, 2H, H2-indole, H-arom); 6.95 (t, 1H, J=7Hz, H-arom); 6.7 (t, 1H, J=7Hz, H-arom); 6.6 (3 equivalent fumaric acid); 6.4-6.5 (2x dd, 2H, J=2Hz, J=8Hz, H-arom); 4.2 (bm, 4H, OCH₂CH₂O); 3.0, 2.7 (2x bs, 8H, piperazine); 2.7 (t, 2H, CH₂); 2.5 (m, 2H, CH₂); 1.7 (m, 2H, CH₂); 1.58 (m, 2H, CH₂); 1.4 (m, 2H, CH₂).

**3-{3-[4-(2,3-Dihydro-benzo[1,4]dioxin-5-yl)-piperazin-1-yl]-propyl}-4-fluoro-1*H-*indole (compound 5d) 0.5 fumaric acid.** Compound **5d** was prepared via route B as a 0.5 fumaric acid salt in an un-optimized yield of 30%. TLC analysis: eluent EtOAc, Rf 0.1; mp 207-208°C; ¹H-NMR (400 MHz, DMSO-d₆): δ 11.0 (s, 1H, NH-indole); 7.15 (d, 1H, J=8Hz, H-arom); 7.07 (d, 1H, J=2Hz, H2-indole); 7.0 (m, 1H, H-arom); 6.62-6.72 (m, 2H, H-arom); 6.6 (s, 0.5 equivalent fumaric acid); 6.4-6.5 (2x dd, 2H, J=2Hz, J=8Hz, H-arom); 4.2 (bm, 4H, OCH₂CH₂O); 3.0, 2.6 (2x bs, 8H, piperazine); 2.8 (t, 2H, J=7Hz, CH₂); 2.5 (m, 2H, CH₂); 1.9 (m, 2H, CH₂).

**3-{3-[4-(2,3-Dihydro-benzo[1,4]dioxin-5-yl)-piperazin-1-yl]-propyl}-5-fluoro-1*H-*indole (compound 5e)** 0.5 fumaric acid. Compound **5e** was prepared via route B as a 0.5 fumaric acid salt in a yield of 70%. TLC analysis: eluent EtOAc, Rf 0.2; ¹H-NMR (400 MHz, DMSO-d₆): δ 10.8 (s, 1H, NH-indole); 7.3(dd, 1H, J=5Hz, J=8Hz, H-arom); 7.2 (dd 1H, J=2Hz, J=8Hz, H-arom); 7.15 (d, 1H, J=2Hz, H2-indole); 6.85 (dt, 1H, J=2Hz, J= 8Hz, H-arom); 6.7 (t, 1H, J=7Hz, H-arom); 6.6 (s, 0.5 equivalent fumaric acid); 6.4-6.5 (2xd, 2H, J=8Hz, H-arom); 4.2 (bm, 4H, OCH₂CH₂O); 3.0, 2.6 (2xd, 8H, piperazine); 2.7 (t, 2H, J=7Hz, CH₂); 2.44 (t, 2H, J=7Hz, CH₂); 1.84 (m, 2H, CH₂).

**3-{3-[4-(2,3-Dihydro-benzo[1,4]dioxin-5-yl)-piperazin-1-yl]-propyl}-6-fluoro-1*H-*indole (compound 5f) 0.5 fumaric acid salt.** Compound **5f** was prepared *via* route B in a yield of 56%. TLC analysis and silica gel column chromatography: eluent EtOAc, Rf 0.3; mp 205-206°C; ¹H-NMR (400 MHz, DMSO-d₆): δ 10.8 (s, 1H, NH-indole); 7.5 (dd 1H, J=5Hz, J=8Hz, H-arom); 7.08 (m, 2H, H2-indole, H-arom); 6.8 (m, 1H, H-arom); 6.7 (t, 1 H, J=7Hz, H-arom); 6.6 (s, 0.5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.7 (t, 2H, CH₂); 2.47 (t, 2H, CH₂); 1.85 (m, 2H, CH₂).

**3-{3-[4-(2,3-Dihydro-benzo[1,4]dioxin-5-yl)-piperazin-1yl]-propyl}-7-fluoro-1*H-*indole (compound 5g) 0.5 fumaric acid salt.** Compound **5g** was prepared *via* route B in a yield of 68%. TLC analysis and silica gel chromatography: eluent EtOAc, Rf 0.25; ¹H-NMR (400 MHz, DMSO-d₆): δ 11.2 (s, 1H, NH-indole); 7.32 (d 1H, J=8Hz, H-arom); 7.14 (d,1H, J=2Hz, H2-indole); 6.9-6.96 (m, 1H, H-arom); 6.82 (dd, 1H, J=8Hz, J=11Hz, H-arom); 6.7 (t, 1 H, J=7Hz, H-arom); 6.6 (s, 0.5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.73 (t, 2H, J=7Hz, CH₂); 2.48(t, 2H, J=7Hz, CH₂); 1.87 (m, 2H, CH₂).

**3-{3-[4-(2,3-Dihydro-benzo[1,4]dioxin-5-yl)-piperazin-1-yl]-propyl}-5-methoxy-1*H*-indole (compound 5h) fumaric acid salt.** Compound **5h** was prepared *via* route B in a yield of 83%; TLC analysis and silica gel chromatography: eluent diethyl ether, Rf 0.15; ¹H-NMR (400 MHz, DMSO-d₆): δ 10.5 (s, 1H, NH-indole); 7.22 (d, 1H, J=9Hz, H-arom); 7.04 (d, 1 H, J=2Hz, H2-indole); 6.96 (d, 1 H, J=2Hz, H-arom); 6.7 (m, 2H, H-arom); 6.6 (s, 2H, fumaric acid); 6.4-6.5 (2x dd, 2H, J=1.5Hz, J=8Hz, H-arom); 4.2 (bm, 4H, OCH₂CH₂O); 3.78 (s, 3H, OMe); 3.04, 2.63 (2x bs, 8H, piperazine); 2.7 (t, 2H, J=7Hz, CH₂); 2.5 (t, 2H, CH₂); 1.87 (q, 2H, CH₂). HRMS (C₂₄H₃₀N₃O₃) [M+H]⁺: found *m*/*z* 408.2292, calculated 408.2292.

**3-{3-[4-(2,3-Dihydro-benzo[1,4]dioxin-5-yl)-piperazin-1-yl]-propyl}-4-chloro-1*H-*indole (compound 5i) 0.5 fumaric acid.** Compound **5i** was prepared *via* route B as a 0.5 fumaric acid salt in a yield of 69%. TLC analysis and silica gel column chromatography: eluent EtOAc, Rf 0.2; ¹H-NMR (400 MHz, DMSO-d₆): δ 11.1 (s, 1H, NH-indole); 7.29 (d, 1H, J=8Hz, H-arom); 7.14 (d,1H, J=2Hz, H2-indole); 7.0 (t, 1H, J=7Hz, H-arom); 6.94 (d, 1 H, J=8Hz, H-arom); 6.7 (t, 1 H, J=7Hz, H-arom); 6.6 (s, 1H, 0,5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.92 (t, 2H, J=7Hz, CH₂); 2.5 (t, 2H, J=7Hz, CH₂); 1.87 (m, 2H, CH₂).

**3-{3-[4-(2,3-Dihydro-benzo[1,4]dioxin-5-yl)-piperazin-1-yl]-propyl}-5-chloro-1*H-*indole (compound 5j) 0.5 fumaric acid salt.** Compound **5j** was prepared via route B in a yield of 62%. Mp 61-63°C (free base); mp 220-222°C (dec) of the 0.5 equivalent fumaric acid; ¹H-NMR (400 MHz, DMSO-d₆): δ11.0 (s, 1H, NH-indole); 7.54 (d, 1H, J=2Hz, H-arom); 7.34 (d, 1H, J=8Hz, H-arom); 7.14 (d,1H, J=2Hz, H2-indole); 7.03 (dd, 1 H, J=2Hz, J=8Hz, H-arom); 6.7 (t, 1 H, J=8Hz, H-arom); 6.6 (s, 1 H, fumaric acid); 6.4-6.5 (2x dd, 2H, J=1.5Hz, J=8Hz, H-arom); 4.2 (bm, 4H, OCH₂CH₂O); 3.06, 2.7 (2x m, 10H, 8H piperazine, CH₂); 2.5 (m, 2H, CH₂); 1.87 (m, 2H, CH₂). HRMS (C₂₃H₂₇ClN₃O₂) [M+H]⁺: found *m*/*z* 412.1798, calculated 412.1792.

**3-{3-[4-(2,3-Dihydro-benzo[1,4]dioxin-5-yl)-piperazin-1-yl]-propyl}-6-chloro-1*H-*indole (compound 5k) 0.5 fumaric acid.** Compound **5k** was prepared *via* route B as a 0.5 fumaric acid salt in a yield of 61%, silica gel column chromatography: eluent EtOAc, Rf 0.25; ¹H-NMR (400 MHz, DMSO-d₆) : δ 10.8 (s, 1H, NH-indole); 7.5 (d, 1H, J=8Hz, H-arom); 7.35 (d, 1H, J=1.5Hz, H-arom); 7.14 (d,1H, J=2Hz, H2-indole); 6.96 (dd, 1H, J=2Hz, J=8Hz, H-arom); 6.7 (t, 1H, J=7Hz, H-arom); 6.6 (s, 1H, 0.5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.72 (t, 2H, J=7Hz, CH₂); 2.46 (t, 2H, J=7Hz, CH₂); 1.85 (m, 2H, CH₂).

**3-{3-[4-(2,3-Dihydro-benzo[1,4]dioxin-5-yl)-piperazin-1-yl]-propyl}-7-chloro-1*H-*indole (compound 51) 0.5 fumaric acid salt.** Compound **5l** was prepared via route B in a yield of 73%, silica gel column chromatography: eluent diethyl ether, Rf 0.1; ¹H-NMR (400 MHz, DMSO-d₆): δ 11.1 (s, 1H, NH-indole); 7.5 (d, 1H, J=8Hz, H-arom); 7.17 (d,1H, J=2Hz, H2-indole); 7.12 (d, 1H, J=8Hz, H-arom); 6.96 (t, 1H, J=7Hz, H-arom); 6.7 (t, 1H, J=7Hz, H-arom); 6.6 (s, 1 H, 0.5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.72 (t, 2H, J=7Hz, CH₂); 2.48 (t, 2H, J=7Hz, CH₂); 1.86 (m, 2H, CH₂).

**3-{3-[4-(2,3-Dihydro-benzo[1,4]dioxin-5-yl)-piperazin-1-yl]-propyl}-5-methyl-1*H-*indole (compound 5m) 0.5 fumaric acid salt.** Compound **5m** was prepared *via* route B in a yield of 65%. Mp free base: 65-67°C; fumaric acid salt: mp 215-217°C (dec); ¹H-NMR (400 MHz, DMSO-d₆): δ10.6 (s, 1H, NH-indole); 7.31 (bs, 1H, H-arom); 7.23 (d,1H, J=8Hz, H-arom); 7.04 (d, 1H, J=2Hz, H2-indole); 6.90 (dd, 1H, J=1.5Hz, J=8Hz, H-arom); 6.74 (t, 1H, J=8Hz, H-arom); 6.6 (s, 1H, 0.5 equivalent fumaric acid); 6.4-6.5 (2x dd, 2H, J=1.5Hz, J=8Hz, H-arom); 4.2 (bm, 4H, OCH₂CH₂O); 3.06, 2.7 (2x m, 10H, CH₂, 8H-piperazine); 2.57 (bm, 2H, CH₂); 2.40 (s, 3H, Me); 1.90 (m, 2H, CH₂). HRMS (C₂₄H₃₀N₃O₂) [M+H]⁺: found *m*/*z* 392.2356, calculated 392.2338.

**3-{3-[4-(2,3-Dihydro-benzo[1,4]dioxin-5-yl)-piperazin-1-yl]-propyl}-7-methyl-1*H-*indole (compound 5n) 0.5 fumaric acid salt.** Compound **5n** was prepared via route B in a yield of 78%. TLC analysis and silica gel column chromatography: eluent EtOAc, Rf 0.3; ¹H-NMR (400 MHz, DMSO-d₆): δ 10.65 (s, 1H, NH-indole); 7.32 (d, 1H, J=8Hz, H-arom); 7.06 (d,1H, J=2Hz, H2-indole); 6.82-6.92 (m, 2H, H-arom); 6.7 (t, 1H, J=7Hz, H-arom); 6.6 (s, 1 H, 0.5 equivalent fumaric acid); 6.4-6.5 (2x dd, 2H, J=1.5Hz, J=8Hz, H-arom); 4.2 (bm, 4H, OCH₂CH₂O); 3.0, 2.6 (2x bs, 8H, piperazine); 2.72 (t, 2H, J=7Hz, CH₂); 2.48 (t, 2H, J=7Hz, CH₂); 2.44 (s, 3H, Me); 1.86 (q, 2H, CH₂). HRMS (C₂₄H₃₀N₃O₂) [M+H]⁺: found *m*/*z* 392.2362, calculated 392.2338.

### Synthesis of compound 14 (according to Scheme 2)

**1-Benzyl-4-(3-prop-2-ynyloxy-phenyl)-piperazine (compound 10).** To a solution of compound 9 (27 g, 90 mmol) in MeOH (200 ml) was added a solution of sodium (4.15 g, 180 mmol) in MeOH (200 ml). The mixture was stirred for 30 minutes at RT and subsequently concentrated *in vacuo*. The residue was dissolved in DMF (200 ml) and a solution of propargyl bromide (11.3 g, 95 mmol) in DMF (50 ml) was added. The mixture was stirred overnight at room temperature. The reaction mixture was quenched with water and the resulting mixture was extracted with DCM (2x300 ml). The combined organic layers were washed with water, dried (MgSO₄) and concentrated to give crude compound 10 as a black oil, which was purified by silica gel column chromatography (DCM/MeOH, 98/2, v/v) to give compound 10 (20 g, 60%) as a colorless oil. ¹H-NMR (400 MHz, DMSO-d₆): δ 7.0-7.4 (m, 6H, H-arom); 6.4-6.6 (m, 3H, H-arom); 4.7 (d, 2H, J=2Hz, O-CH₂); 3.5 (s, 2H, N-CH₂); 3.1, 2.5 (2x m, 8H, piperazine); 1.98 (s, H, CH).

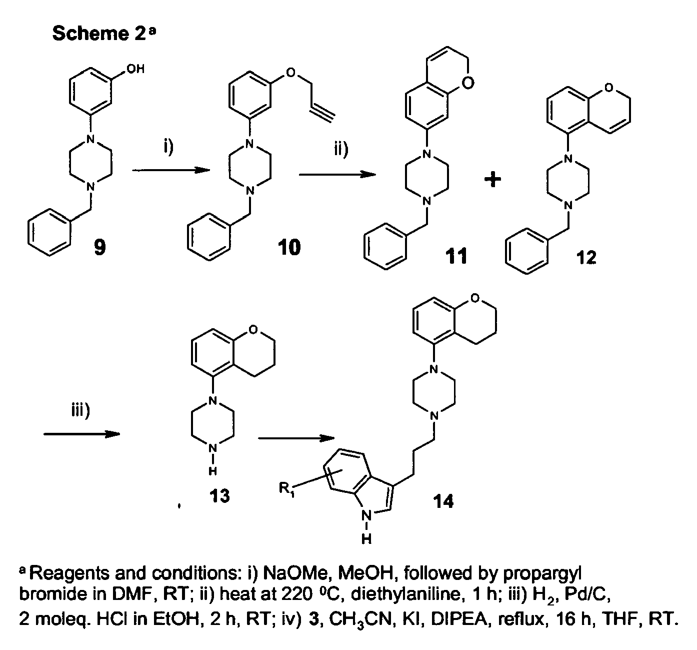

**1-Benzyl-4-(2*H*-chromen-5-yl)-piperazine (compound 12).** Compound **10** was dissolved in diethyl aniline (45 ml) and the solution was heated at 220°C for 1 hour, after which time TLC analysis revealed complete conversion of compound 10 into two new products. The reaction mixture was allowed to cool to room temperature and concentrated *in vacuo* to give a brown oil, which was purified by silica gel column chromatography to give pure compound 12 (10.6 g, 69%) and compound 11 (3.4 g, 22%) as colourless oils. Compound 11: ¹H-NMR (400 MHz, CDCl₃): δ 7.3-7.4 (m, 5H, phenyl); 6.84 (d,1H, J=8Hz, H-arom); 6.42 (dd, 1H, J=2Hz, J=8Hz, H-arom); 6.32-6.38 (m, 2H, H-4, H-arom); 5.6 (m, 1H, H3); 4.76 (dd, 2H, H-2); 3.54 (s, 2H, CH₂ Bn ); 3.0, 2.6 (2x m, 8H, piperazine). Compound 12: ¹H-NMR (400 MHz, CDCl₃): δ 7.24-7.4 (m, 5H, phenyl); 7.06 (t,1H, J=8Hz, H-arom); 6.7 (m, 1H, J=2Hz, J=8Hz, H-4); 6.55 (d, 2H, J=8Hz, H-arom); 5.7-5.8 (dt, 1H, J=3Hz, J=8Hz, H-3); 4.66 (dd, 2H, J=2Hz, J=4Hz, H-2); 3.6 (s, 2H, CH₂Bn); 3.0, 2.6 (2x m, 8H, piperazine).

**1-Chroman-5-yl-piperazine (compound 13).** Compound **12** (2.4 g, 8mmol) was dissolved in ethanol containing hydrochloric acid (2 mol. equivalent) and the catalyst Pd on charcoal (100 mg) was added. The mixture was shaken for 2 hours under an atmosphere of hydrogen. The mixture was filtered and concentrated *in vacuo* to give crude compound 13 as an oil, which was purified by silica gel column chromatography to give pure compound 13 (2 g, 85%) as the hydrochloric acid salt.; ¹H-NMR (400 MHz, CDCl₃): δ 7.2 (t,1H, J=8Hz, H-b); 6.7-6.8 (2xd, 2H, J=8Hz, H5, H7); 4.2 (t, 2H, J=5Hz, H2); 3.4, 3.2 (2x m, 8H, piperazine); 2.8 (t, 2H, H4); 2.0 (m, 2H, H3).

**3-[3-(4-chroman-5-yl-piperazine-1-yl)-propyl]-1*H*-indole (compound 14) 0.5 fumaric acid salt.** Compound 13 (1.75 g, 6mmol) was converted with compound 3b *via* route B to give compound **14** (1.4 g, 50%) as a white solid. TLC analysis and silica gel column chromatography: eluent: DMA 0.5 Rf 0.7; ¹H-NMR (400 MHz, DMSO-d₆): δ 10.7 (s, 1H, NH-indole); 7.5 (d, 1H, J=8Hz, H-arom); 7.32 (d, 1H, J=8Hz, H-arom); 7.06 (d, 1H, J=2Hz, H2-indole); 7.04 (t, 1 H, J=7Hz, H-arom); 6.9-7.0 (m, 2H, H-arom); 6.6 (s, 1H, 0.5 fumaric acid); 6.5 (d, 1 H, J=8Hz, H-arom); 6.44 (d, 1H, J=8Hz, H-arom); 4.1 (t, 2H, OCH₂); 2.9, 2.6 (2x bs, 8H, piperazine); 2.72 (t, 2H, CH₂); 2.64, 2.5 (2xt, 4H, CH₂); 1.9 (m, 4H, H-2, CH₂).

**Table 1. Syntheses of compounds with general formula (1).**

| | | | | | | | | | | | |
|---|---|---|---|---|---|---|---|---|---|---|---|
| 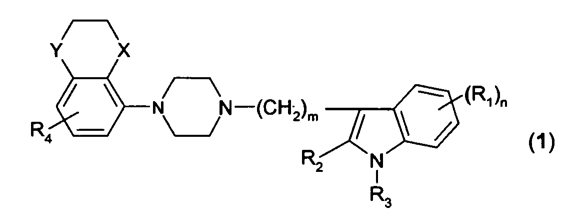 | | | | | | | | | | | |

| **No** | **m** | **n** | **R₁** | **R** | **R₃** | **R₄** | **Y** | **X** | **Rt** | **Salt** | **Yield** |
|---|---|---|---|---|---|---|---|---|---|---|---|
| | | | | | | | | | | | |
| **5a** | 1 | 0 | - | H | H | H | ○ | ○ | - | - | 33% |
| **5b** | 3 | 0 | - | H | H | H | ○ | ○ | B | - | 77% |
| **5c** | 5 | 0 | - | H | H | H | ○ | ○ | A | 0.5 fum | 35% |
| **5d** | 3 | 1 | 4-F | H | H | H | ○ | ○ | B | 0.5 fum | 30% |
| **5e** | 3 | 1 | 5-F | H | H | H | ○ | ○ | B | 0.5 fum | 70% |
| **5f** | 3 | 1 | 6-F | H | H | H | ○ | ○ | B | 0.5 fum | 56% |
| **5g** | 3 | 1 | 7-F | H | H | H | ○ | ○ | B | 0.5 fum | 68% |
| **5h** | 3 | 1 | 5-OCH₃ | H | H | H | ○ | ○ | B | 0.5 fum | 83% |
| **5i** | 3 | 1 | 4-Cl | H | H | H | ○ | ○ | B | 0.5 fum | 69% |
| **5j** | 3 | 1 | 5-Cl | H | H | H | ○ | ○ | B | 0.5 fum | 62% |
| **5k** | 3 | 1 | 6-Cl | H | H | H | ○ | ○ | B | 0.5 fum | 61% |
| **5l** | 3 | 1 | 7-Cl | H | H | H | ○ | ○ | B | 0.5 fum | 73% |
| **5m** | 3 | 1 | 5- CH₃ | H | H | H | ○ | ○ | B | 0.5 fum | 65% |
| **5n** | 3 | 1 | 7- CH₃ | H | H | H | ○ | ○ | B | 0.5 fum | 78% |
| **14** | 3 | 0 | - | H | H | H | ○ | CH₂ | - | - | 50% |

| | | | | | | | | | | | |
|---|---|---|---|---|---|---|---|---|---|---|---|
| ***No** = compound number as used in te xt, **Rt*** = *Route A or B as depeicted in Scheme 1;* *fum = fumarate; percentage yield is based on compound 2.* | | | | | | | | | | | |

The specific compounds of which the synthesis is described above are intended to further illustrate the invention in more detail, and therefore are n ot deemed to restrict the scope of the invention in any way. Other embodiments of the invention will be apparent to those skilled in the art from consideration of the specification and practice of the invention disclosed herein. It is thus intended that th e specification and examples be considered as exemplary only, with a true scope and spirit of the invention being indicated by the claims.

### EXAMPLE 2: FORMULATION OF COMPOUND 5e USED IN BIOASSAYS

For oral **(*p*.*o*.)** administration : to the desired quantity (0.5-5 mg) of the solid compound 5e in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1N). Remaining particles in the suspension were further suspended by using an ultrasonic bath.

**For intraperitoneal (*i*.*p*.) administration :** to the desired quantity (0.5-15 mg) of the solid compound 5e in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 3: PHARMACOLOGICAL TESTRESULTS

**Table 2. In vitro and in vivo test results.**

| The results in the table below were obtained according to the methods described above. Binding data are average values from three independent experiments. | | | | |
|---|---|---|---|---|
| | *in vitro affinity* | | *in vivo activity* | |
| | | | | |
| No | Dopamine-D₂ | 5-HT reuptake | Dopamine-D₂ | 5-HT reuptake |
| | | | | |
| | binding | binding | APO-climb* | 5-HTP** |
| | | | | |
| No | Kᵢ(nM) | Kᵢ(nM) | ED₅₀ mg/kg po | ED₅₀ mg/kg po |
| | | | | |
| 5a | 62.0 ± 17 | 176.0 ± 59 | - | - |
| 5b | 21.0 ± 9.9 | 0.4 ± 0.1 | 10 | 22 |
| 5c | 9.1 ± 1.8 | 37.0 ± 3 | - | - |
| 5d | 8.1 ± 3.1 | 0.4 ± 0.1 | 13 | 32 |
| 5e | 19.0 ± 3 | 0.3 ± 0.1 | 15 | 2.0 |
| 5f | 14.6 ± 3.5 | 1.6 ± 0.2 | 9 | 30 |
| 5g | 10.6 ± 4 | 0.6 ± 0.1 | 4.1 | 28.4 |
| 5h | 5.0 ± 4 | 14.0 ± 4 | - | - |
| 5i | 24.0 ± 3 | 1.4 ± 0.4 | - | - |
| 5j | 31.6 ± 16 | 1.4 ± 0.7 | - | - |
| 5k | 2.1 ± 1 | 2.0 ± 0.7 | 4.5 | 86 |
| 5l | 15.5 ± 5.4 | 7.9 ± 2.8 | - | - |
| 5m | 10.7 ± 2.8 | 1.3 ± 0.9 | - | - |
| 5n | 15.9 ± 4.7 | 12.0 ± 5 | - | - |
| 14 | 20.3 ± 2.7 | 1.1 ± 0.3 | - | - |

## Claims

1. Compounds of the general formula (1): 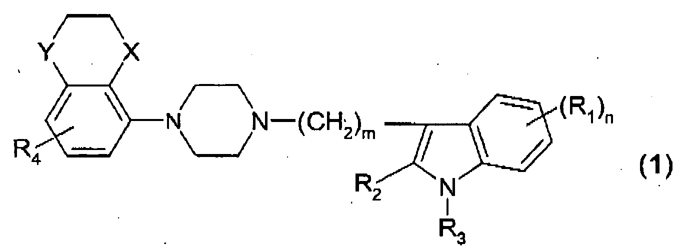 wherein:
Y is O, X is CH₂ or O, m is 3 or 5, n is 1, R₂, R₃ and R₄ are hydrogen, and R₁ is hydrogen, methyl, fluorine, chlorine or methoxy, and tautomers, stereo-isomers and N-oxides thereof, as well as pharmacologically acceptable salts, hydrates and solvates of said compounds of formula (1) and its tautomers, stereoisomers and N-oxides.

2. A compound as claimed in claim 1, selected from the group:
| **m** | **n** | **R₁** | **R₂** | **R₃** | **R₄** | **Y** | **X** |
|---|---|---|---|---|---|---|---|
| | | | | | | | |
| 1 | 0 | - | H | H | H | ○ | ○ |
| 3 | 0 | - | H | H | H | ○ | ○ |
| 5 | 0 | - | H | H | H | ○ | ○ |
| 3 | 1 | 4-F | H | H | H | ○ | ○ |
| 3 | 1 | 5-F | H | H | H | ○ | ○ |
| 3 | 1 | 6-F | H | H | H | ○ | ○ |
| 3 | 1 | 7-F | H | H | H | ○ | ○ |
| 3 | 1 | 5-OCH₃ | H | H | H | ○ | ○ |
| 3 | 1 | 4-Cl | H | H | H | ○ | ○ |
| 3 | 1 | 5-Cl | H | H | H | ○ | ○ |
| 3 | 1 | 6-Cl | H | H | H | ○ | ○ |
| 3 | 1 | 7-Cl | H | H | H | ○ | ○ |
| 3 | 1 | 5- CH₃ | H | H | H | ○ | ○ |
| 3 | 1 | 7- CH₃ | H | H | H | ○ | ○ |
| 3 | 0 | - | H | H | H | ○ | CH₂ |
wherein the symbols represent those in formula (1): 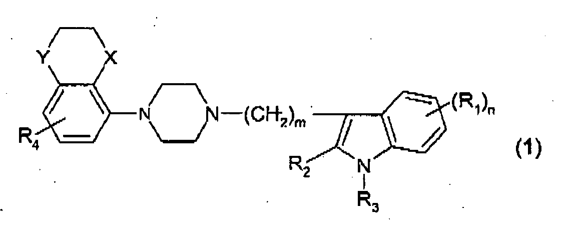 and tautomers, stereoisomers and N-oxides thereof, as well as pharmacologically acceptable salts, hydrates and solvates of said compounds of formula (1) and its tautomers, stereoisomers and N-oxides.

3. A pharmaceutical composition comprising, in addition to a pharmaceutically acceptable carrier and/or at least one pharmaceutically acceptable auxiliary substance, a pharmacologically active amount of at least one compound of one of the claims 1 or 2, or a salt thereof, as an active ingredient.

4. A method of preparing a composition as claimed in claim 3, **characterised in that** at least one compound of one of the claims 1 or 2, or a salt thereof, is brought into a form suitable for administration.

5. A compound as claimed in any of the claims 1 or 2, or a salt thereof, for use as a medicament.

6. Use of a compound as claimed in any of the claims 1 or 2 for the preparation of a pharmaceutical composition for the treatment of disorders of the central nervous system.

7. Use as claimed in claim 6, **characterized in that** said disorders are aggression, anxiety disorders, autism, vertigo, depression, disturbances of cognition or memory, Parkinson's disease, schizophrenia and other psychotic disorders.

8. Use as claimed in claim 6, **characterized in that** said disorder is depression.

9. Use as claimed in claim 6, **characterized in that** said disorders are schizophrenia and other psychotic disorders.

## Patentansprüche

1. Verbindungen der allgemeinen Formel (1): 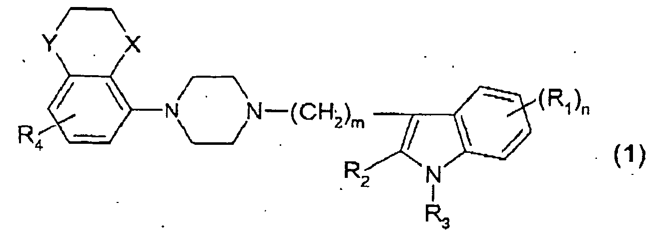 worin:
Y für O steht, X für CH₂ oder O steht, m 3 oder 5 ist, n 1 ist, R₂, R₃ und R₄ Wasserstoff darstellen und R₁ Wasserstoff, Methyl, Fluor, Chlor oder Methoxy darstellt, und Tautomere, Stereoisomere und N-Oxide davon, als auch pharmakologisch annehmbare Salze, Hydrate und Solvate von besagten Verbindungen der Formel (1) und ihren Tautomeren, Stereoisomeren und N-Oxiden.

2. Verbindung wie in Anspruch 1 beansprucht, ausgewählt aus der Gruppe:
| **m** | **n** | **R₁** | **R₂** | **R₃** | **R₄** | **Y** | **X** |
|---|---|---|---|---|---|---|---|
| 1 | 0 | - | H | H | H | ○ | ○ |
| 3 | 0 | - | H | H | H | ○ | ○ |
| 5 | 0 | - | H | H | H | ○ | ○ |
| 3 | 1 | 4-F | H | H | H | ○ | ○ |
| 3 | 1 | 5-F | H | H | H | ○ | ○ |
| 3 | 1 | 6-F | H | H | H | ○ | ○ |
| 3 | 1 | 7-F | H | H | H | ○ | ○ |
| 3 | 1 | 5-OCH₃ | H | H | H | ○ | ○ |
| 3 | 1 | 4-Cl | H | H | H | ○ | ○ |
| 3 | 1 | 5-Cl | H | H | H | ○ | ○ |
| 3 | 1 | 6-Cl | H | H | H | ○ | ○ |
| 3 | 1 | 7-Cl | H | H | H | ○ | ○ |
| 3 | 1 | 5-CH₃ | H | H | H | ○ | ○ |
| 3 | 1 | 7-CH₃ | H | H | H | ○ | ○ |
| 3 | 0 | - | H | H | H | O | CH₂ |
worin die Symbole jene in Formel (1) darstellen: 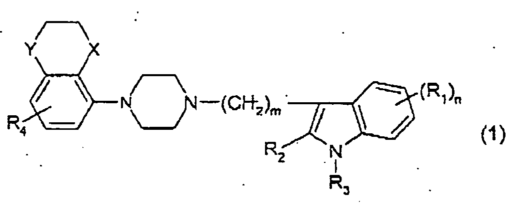 und Tautomere, Stereoisomere und N-Oxide davon, als auch pharmakologisch annehmbare Salze, Hydrate und Solvate von besagten Verbindungen der Formel (1) und ihren Tautomeren, Stereoisomeren und N-Oxiden.

3. Pharmazeutische Zusammensetzung, welche zusätzlich zu einem pharmazeutisch annehmbaren Träger und/oder mindestens einer pharmazeutisch annehmbaren Hilfssubstanz eine pharmakologisch wirksame Menge von mindestens einer Verbindung nach einem der Ansprüche 1 oder 2 oder ein Salz davon als wirksamen Inhaltsstoff umfasst.

4. Verfahren zum Herstellen einer Zusammensetzung wie in Anspruch 3 beansprucht, **dadurch gekennzeichnet, dass** mindestens eine Verbindung von einem der Ansprüche 1 oder 2 oder ein Salz davon in eine für Verabreichung geeignete Form gebracht wird.

5. Verbindung wie in einem der Ansprüche 1 oder 2 beansprucht oder ein Salz davon zur Verwendung als Medikament.

6. Verwendung einer Verbindung wie in einem der Ansprüche 1 oder 2 beansprucht für die Herstellung einer pharmazeutischen Zusammensetzung für die Behandlung von Störungen des zentralen Nervensystems.

7. Verwendung wie in Anspruch 6 beansprucht, **dadurch gekennzeichnet, dass** besagte Störungen Aggression, Angststörungen, Autismus, Schwindel, Depression, Störungen von Kognition oder Gedächtnis, Parkinson-Krankheit, Schizophrenie und andere psychotische Störungen sind.

8. Verwendung wie in Anspruch 6 beansprucht, **dadurch gekennzeichnet, dass** besagte Störung Depression ist.

9. Verwendung wie in Anspruch 6 beansprucht, **dadurch gekennzeichnet, dass** besagte Störungen Schizophrenie und andere psychotische Störungen sind.

## Revendications

1. Composés de formule générale (1) : 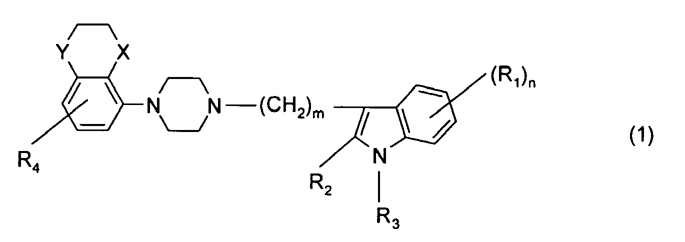 où :
Y est O, X est CH₂ ou O, m est 3 ou 5, n est 1, R₂, R₃ et R₄ sont l'hydrogène, et R₁ est l'hydrogène, méthyle, le fluor, le chlore ou méthoxy et leurs tautomères, stéréoisomères et N-oxydes, ainsi que les sels pharmacologiquement acceptables, hydrates et solvates desdits composés de formule (1) et leurs tautomères, stéréoisomères et N-oxydes.

2. Composé selon la revendication 1 choisi dans le groupe :
| m | n | R₁ | R₂ | R₃ | R₄ | Y | X |
|---|---|---|---|---|---|---|---|
| 1 | ○ | - | H | H | H | ○ | ○ |
| 3 | ○ | - | H | H | H | ○ | ○ |
| 5 | ○ | - | H | H | H | ○ | ○ |
| 3 | 1 | 4-F | H | H | H | ○ | ○ |
| 3 | 1 | 5-F | H | H | H | ○ | ○ |
| 3 | 1 | 6-F | H | H | H | ○ | ○ |
| 3 | 1 | 7-F | H | H | H | ○ | ○ |
| 3 | 1 | 5-OCH₃ | H | H | H | ○ | ○ |
| 3 | 1 | 4-Cl | H | H | H | ○ | ○ |
| 3 | 1 | 5-Cl | H | H | H | ○ | ○ |
| 3 | 1 | 6-Cl | H | H | H | ○ | ○ |
| 3 | 1 | 7-Cl | H | H | H | ○ | ○ |
| 3 | 1 | 5-CH₃ | H | H | H | ○ | ○ |
| 3 | 1 | 7-CH₃ | H | H | H | ○ | ○ |
| 3 | 0 | - | H | H | H | ○ | CH₂ |
où les symboles représentent ceux de la formule (1) : 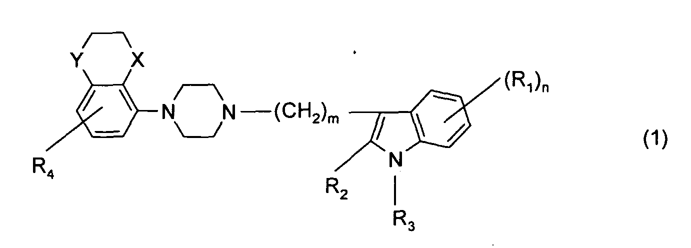 et ses tautomères, stéréoisomères et N-oxydes, ainsi que les sels pharmacologiquement acceptables, hydrates et solvates desdits composés de formule (1) et leurs tautomères, stéréoisomères et N-oxydes.

3. Composition pharmaceutique comprenant, en plus d'un vecteur pharmaceutiquement acceptable et/ou d'au moins une substance auxiliaire pharmaceutiquement acceptable, une quantité pharmacologiquement active d'au moins un composé selon l'une des revendications 1 ou 2, ou d'un sel de celui-ci, comme ingrédient actif.

4. Procédé de préparation d'une composition selon la revendication 3, **caractérisé en ce qu'**au moins un composé selon l'une des revendications 1 ou 2, ou un sel de celui-ci, est mis sous une forme appropriée pour l'administration.

5. Composé selon l'une quelconque des revendications 1 ou 2, ou sel de celui-ci, destiné à être utilisé comme médicament.

6. Utilisation d'un composé selon l'une quelconque des revendications 1 ou 2 pour la préparation d'une composition pharmaceutique pour le traitement des troubles du système nerveux central.

7. Utilisation selon la revendication 6 **caractérisée en ce que** lesdits troubles sont l'agression, les troubles de type anxiété, l'autisme, le vertige, la dépression, les perturbations de la cognition ou de la mémoire, la maladie de Parkinson, la schizophrénie et d'autres troubles psychotiques.

8. Utilisation selon la revendication 6 **caractérisée en ce que** ledit trouble est la dépression.

9. Utilisation selon la revendication 6 **caractérisée en ce que** lesdits troubles sont la schizophrénie et d'autres troubles psychotiques.
